# EUROPEAN PATENT APPLICATION

(11) **EP 3 854 378 A1**
(43) Date of publication of application: **28.07.2021**
(21) Application number: 19859650.4
(22) Date of filing: 06.09.2019
(51) Int. Cl.: A61K 8/81, A61K 8/00, A61K 8/04, A61K 8/19, A61K 8/25, A61K 8/31, A61K 8/34, A61K 8/36, A61K 8/37, A61K 8/45, A61K 8/86, A61K 8/89

(54) **WATER-IN-OIL EMULSION COSMETIC COMPOSITION**

(30) Priority: 14.09.2018 JP 2018173046
(71) Applicant: Shiseido Company, Ltd., Chuo-ku Tokyo 104-0061 (JP)
(72) Inventor: MATSUO Ayano, Tokyo 104-0061 (JP); SOGABE Atsushi, Tokyo 104-0061 (JP); OSAWA Tomo, Tokyo 104-0061 (JP); HITOMI Mao, Tokyo 104-0061 (JP); IKEDA Tomoko, Tokyo 104-0061 (JP); WEN Chunming, Tokyo 104-0061 (JP)
(74) Representative: Ter Meer Steinmeister & Partner
(86) International application number: PCT/JP2019/035123
(87) International publication number: WO 2020/054590

(57) **Abstract**

An object of the present invention is to provide a water-in-oil emulsified cosmetic having improved feeling on use and usability.

A water-in-oil emulsified cosmetic characteristic by comprising the following components (a) to (d):
(a) one or more polymers selected from the group consisting of polyacrylic acid, a salt of polyacrylic acid, poly(2-acrylamido-2-methylpropanesulfonic acid), and a salt of poly(2-acrylamido-2-methylpropanesulfonic acid) and
having a weight average molecular weight of 1 million to 4 million, and
a viscosity at 25°C when made into a 1% by mass aqueous solution of 1 Pa·s or less,
(b) an aqueous phase component other than the component (a)
(c) 60% by mass or less of an oily component, and
(d) an emulsifier.

## Description

### [Related Application]

The present application claims the priority of Japanese Patent Application No. 2018-173046 filed on September 14, 2018, which is incorporated herein.

### FIELD OF THE INVENTION

The present invention relates to a blended water-in-oil emulsified cosmetic into which a molecular weight controlled water-soluble polymer is blended.

### BACKGROUND OF THE INVENTION

The water-soluble synthetic polymer greatly contributes to the viscosity regulation of a composition having water or an aqueous solvent as a continuous phase. In particular, polyacrylic acid or a salt thereof, or poly(2-acrylamido-2-methylpropanesulfonic acid) (hereinafter, sometimes abbreviated as PAMPS) or a salt thereof is widely used as a thickener in water-based and oil-in-water compositions in various fields because of the high water solubility thereof.

However, these water-soluble synthetic polymers exhibit not only a thickening action but also a thread-forming property (spinnability), and thus there is a situation where the amount thereof blended is limited in a composition for which spinnability should be avoided (for example, a cosmetic).

In response to this problem, the present inventor found that a polyacrylate and the like available on the market exhibit spinnability because of the effect of macromolecules (specifically, a molecule having a molecular weight of 10 million or more) mixed therein (Patent Literature 1). These macromolecules result from overpolymerization in the synthesis process and are unavoidable by-products when synthesized by a usual method in which the polymerization initiation frequency and the polymerization rate cannot be sufficiently controlled.

The present inventor made it clear that for a polyacrylate and a PAMPS salt in which the content of the macromolecules is 10% by mass or less by synthesizing the salt by a RAFT polymerization method, the spinnability alone is greatly reduced while the original thickening effect is maintained. Then, the present inventor reported that by using the molecular weight controlled polymer as a thickener instead of the conventional (commercially available) polymer, water-based and oil-in-water emulsified cosmetics that do not feel spinnable can be produced (Patent Literature 1).

However, the above is the effect when a molecular weight controlled polyacrylic acid or a salt thereof or the like is blended into a "system in which the aqueous phase is a continuous phase," and the effect when such an acid or a salt thereof is blended into the inner aqueous phase of a "system in which the oil phase is a continuous phase", that is, a water-in-oil emulsified composition, is completely unknown.

### [Citation List]

### [Patent Literature]

[Patent Literature 1] WO publicated literature No.2015/052804

### DISCLOSURE OF THE INVENTION

### PROBLEM TO BE SOLVED BY THE INVENTION

An object of the present invention is to provide a water-in-oil emulsified cosmetic having improved feeling on use and usability.

### MEANS TO SOLVE THE PROBLEM

As a result of diligent and extensive research by the present inventor on the problem, the present inventor has found that when a molecular weight controlled polyacrylate is blended into the inner aqueous phase of a water-in-oil emulsified cosmetic in which the amount of an oily component blended is 60% or less, the spread is very good, the sticky feeling decreases, the rich feeling increases, and the effect of giving moisturization and smoothness to the skin is far superior, and completed the present invention.

Accordingly, the oil-based cosmetic according to the present invention comprises the following:
[1] A water-in-oil emulsified cosmetic comprising the following components (a) to (d):
   (a) one or more polymers selected from the group consisting of polyacrylic acid, a salt of polyacrylic acid, poly(2-acrylamido-2-methylpropanesulfonic acid), and a salt of poly(2-acrylamido-2-methylpropanesulfonic acid) and
      having a weight average molecular weight of 1 million to 4 million, and
      a viscosity at 25°C when made into a 1% by mass aqueous solution of 1 Pa·s or less,
   (b) an aqueous phase component other than the component (a)
   (c) 60% by mass or less of an oily component, and
   (d) an emulsifier.
[2] The water-in-oil emulsified cosmetic according to [1], wherein a content of the component (a) is 0.005% to 2%.
[3] The water-in-oil emulsified cosmetic according to [1] or [2], wherein a weight average molecular weight of the component (a) is 1.5 million to 4 million.
[4] The water-in-oil emulsified cosmetic according to any one of [1] to [3], further comprising a hydrophobized powder as a component (e).

### EFFECT OF THE INVENTION

According to the present invention, a water-in-oil emulsified cosmetic having very good spreadability, less sticky feeling, good rich feeling, and excellent effect of giving moisturization and smoothness to the skin is provided.

### BEST MODE FOR CARRYING OUT THE INVENTION

A preferred embodiment of the present invention will be described.

In the water-in-oil emulsified cosmetic according to the present invention, the aqueous phase composed of the component (a) and the component (b) is dispersed as an emulsified particle in the oil phase containing the component (c) by the action of the component (d). Hereinafter, each component will be described in detail.

### (a) Component

The component (a) in the present invention is one or more polymers selected from the group consisting of polyacrylic acid, a salt of polyacrylic acid, poly(2-acrylamido-2-methylpropanesulfonic acid), and a salt of poly(2-acrylamido-2-methylpropanesulfonic acid) and having a weight average molecular weight of 1 million to 4 million and a viscosity at 25°C when made into a 1% by mass aqueous solution of 1 Pa·s or less. In addition, the component (a) of the present invention is a linear polymer.

The requirement that "a viscosity at 25°C when made into a 1% by mass aqueous solution of 1 Pa·s or less" means that "only 10% by mass or less of a molecular species having a molecular weight of 10 million or more is contained" in the polymer. This is because a linear polymer having a weight average molecular weight of 1 million to 4 million has a viscosity at 25°C when made into a 1% by mass aqueous solution of 1 Pa·s or less, if the content of a molecular species having a molecular weight of 10 million or more is 10% by mass or less.

The viscosity is an apparent viscosity value after 60 seconds at a strain rate of 10 s⁻¹ measured using a stress controlled rheometer. As the stress controlled rheometer, a cone plate type geometry can be preferably used.

Herein, a "linear polymer having a weight average molecular weight of 1 million to 4 million and a viscosity at 25°C when made into a 1% by mass aqueous solution of 1 Pa·s or less" is sometimes referred to as a "molecular weight controlled polymer." Then, polyacrylic acid and poly(2-acrylamido-2-methylpropanesulfonic acid) that satisfy the requirement of the molecular weight controlled polymer are sometimes referred to as "molecular weight controlled polyacrylic acid" and "molecular weight controlled PAMPS," respectively. Here, "PAMPS" is an abbreviation for poly(2-acrylamido-2-methylpropanesulfonic acid).

The weight average molecular weight of the component (a) according to the present invention is 1 million to 4 million, preferably 1.5 million to 4 million, more preferably 2 million to 3.5 million, and further preferably 2 million to 3 million. If the weight average molecular weight is less than 1 million, an appropriate rich feeling may not be obtained, and if the weight average molecular weight is more than 4 million, stickiness may occur.

Examples of the types of the salts include an alkali metal salt (for example, a sodium salt, a potassium salt, a magnesium salt, or a calcium salt), an organic amine salt (for example, a monoethanolamine salt, a diethanolamine salt, a triethanolamine salt, or a triisopropanolamine salt), and a salt of a basic nitrogen-containing compound such as 2-amino-2-methyl-1-propanol, 2-amino-2-methyl-1,3-propanediol, 2-amino-2-hydroxymethyl-1,3-propanediol, L-arginine, L-lysine, or L-alkyltaurine. Of these, a monovalent alkali metal salt and an organic amine salt are preferable, and a sodium salt, a potassium salt, a triethanolamine salt are more preferable, and a sodium salt is the most preferable.

In the present invention, a polyacrylate or a PAMPS salt means a compound obtained by neutralizing polyacrylic acid or PAMPS with the base (that is, the alkali metal, organic amine, basic nitrogen-containing compound, and the like), or a compound obtained by polymerizing acrylic acid or 2-acrylamido-2-methylpropanesulfonic acid (hereinafter abbreviated as AMPS) whose acid moiety has been neutralized in advance with the base.

### • Method for synthesizing molecular weight controlled polymer

The component (a) according to the present invention can be synthesized by a known living polymerization method. Examples of living polymerization include living anionic polymerization, living cationic polymerization, and living radical polymerization (precision radical polymerization or controlled radical polymerization).

Examples of the living radical polymerization include (radical) polymerization via nitroxide, nitroxide-mediated (radical) polymerization (NLRP), atom transfer radical polymerization (ATRP), and reversible addition-cleavage chain transfer (RAFT) polymerization. Examples of the atom transfer radical polymerization (ATRP) include electron transfer-derived activator ATRP, activators generated by electron transfer ATRP (AGET ATRP), electron transfer-derived regeneration activator ATRP, or activators regenerated by electron transfer ATRP (ARGET ATRP), initiators for continuous active species regeneration ATRP, initiators for constant activator regeneration ATRP (ICAR ATRP), and Reverse ATRP. Examples of a derivative technology of the reversible addition-cleavage chain transfer (RAFT) polymerization include living radical polymerization with organotellurium as the growing end, organotellurium-mediated living radical polymerization (TERP), antimony-mediated living radical polymerization (SBRP), and bismuth-mediated living radical polymerization (BIRP). Other examples of the living radical polymerization include iodine transfer radical polymerization (IRP) and cobalt-mediated radical polymerization (CMRP).

Direct polymerization of acrylic acid is preferable because of the simplicity of polymerization thereof, but if polymerization is difficult because of the formation of an insoluble salt such as a catalyst, a protected acrylate such as t-butyl acrylate, methoxymethyl acrylate, and methyl acrylate can be used followed by deprotection to obtain a polymer compound of interest.

In the present invention, the reversible addition-cleavage chain transfer polymerization method (RAFT polymerization method) is particularly preferable in that the molecular weight control of the polymer (that is, the synthesis of a polymer compound having a narrow molecular weight distribution) is possible (Patent Literature 4). The chain transfer agent is preferably a dithio type or a trithio type. The polymerization initiator preferably has a chemical structure similar to that of the chain transfer agent, and an azo-based initiator is preferable. The polymerization solvent is not particularly limited, and one having high solubility in a monomer and a polymer is appropriately selected. The polymerization time is preferably about several hours to 100 hours.

### • Molecular weight measurement method

For the molecular weight of the molecular weight controlled polymer, the weight average molecular weight can be measured by a known method such as a light scattering method, an ultracentrifugation method, or a chromatographic method, and the number average molecular weight can be measured by a known method such as an osmotic pressure method or a chromatographic method. Of these, the chromatographic method is preferable in that the weight average molecular weight, the number average molecular weight, and the molecular weight distribution can be easily obtained using a small amount of a sample, and a gel permeation chromatographic method (hereinafter abbreviated as GPC) is further preferable.

The molecular weight distribution used in the present application is a value obtained by dividing the weight average molecular weight obtained by GPC analysis by the number average molecular weight.

The amount of the component (a) blended in the water-in-oil emulsified cosmetic according to the present invention is 0.005 to 2% by mass, preferably 0.005 to 1.5% by mass, and more preferably 0.005 to 1% by mass. If the amount blended is less than 0.005% by mass, an appropriate rich feeling may not be obtained, and if the amount blended exceeds 2% by mass, stickiness may occur.

### (b) Aqueous phase component other than component (a)

In the present invention, as an aqueous phase component other than the component (a), in addition to a water-based solvent such as water, an aqueous component usually used in a cosmetic product can be blended in a range that does not impair the effects of the present invention.

Examples of the water-based solvent include water and a water-soluble alcohol.

As water, purified water such as distilled water, reverse osmosis water, and ion-exchanged water, tap water, and the like can be used.

Examples of the water-soluble alcohol include a lower alcohol, a polyhydric alcohol, a polyhydric alcohol polymer, a dihydric alcohol alkyl ether, a dihydric alcohol alkyl ether, a dihydric alcohol ether ester, a glycerin monoalkyl ether, and a sugar alcohol.

### Examples of the lower alcohol include ethanol, propanol, isopropanol, isobutyl alcohol, and t-butyl alcohol

Examples of the polyhydric alcohol include a dihydric alcohol (for example, dipropylene glycol, 1,3-butylene glycol, ethylene glycol, trimethylene glycol, 1,2-butylene glycol, tetramethylene glycol, 2,3-butylene glycol, pentamethylene glycol, 2-butene-1,4-diol, hexylene glycol, or octylene glycol), a trihydric alcohol (for example, glycerin or trimethylolpropane), a tetrahydric alcohol (for example, diglycerin or a pentaerythritol such as 1,2,6-hexanetriol), a pentahydric alcohol (for example, xylitol or triglycerin), a hexahydric alcohol (for example, sorbitol or mannitol), a polyhydric alcohol polymer (for example, diethylene glycol, dipropylene glycol, triethylene glycol, polypropylene glycol, tetraethylene glycol, diglycerin-triglycerin, tetraglycerin, or polyglycerin), and a dihydric alcohol alkyl ether (for example, ethylene glycol monomethyl ether, ethylene glycol monoethyl ether, ethylene glycol monobutyl ether, ethylene glycol monophenyl ether, ethylene glycol monohexyl ether, ethylene glycol mono2-methylhexyl ether, ethylene glycol isoamyl ether, ethylene glycol benzyl ether, ethylene glycol isopropyl ether, ethylene glycol dimethyl ether, ethylene glycol diethyl ether, or ethylene glycol dibutyl ether).

Examples of the aqueous component include a moisturizer, a water-soluble polymer, a hydrophilic powder, an inorganic salt, an organic salt, a vitamin, and a pH adjuster.

Examples of the moisturizer include 1,3-butylene glycol, polyethylene glycol, propylene glycol, dipropylene glycol, hexylene glycol, glycerin, diglycerin, xylitol, maltitol, maltose, and D-mannit. Furthermore, a polyoxyethylene (hereinafter, POE)/polyoxypropylene (hereinafter, POP) copolymer dialkyl ether can also be blended. Each alkyl group of the POE/POP copolymer dialkyl ether may be the same or different alkyl group having 1 to 4 carbon atoms, preferably a methyl group or an ethyl group, and particularly preferably a methyl group. The POE group is preferably 20 to 80% by weight based on the total of the POE group and the POP group. The POE/POP may be either a random type or a block type, and is preferably a random type. Such a POE/POP copolymer dialkyl ether is described in, for example, Japanese Unexamined Patent Literature 2004-83541 and Japanese Japanese Unexamined Patent Literature 2006-265135. Examples of such a compound include POE (9) POP (2) dimethyl ether, POE (7) POP (12) dimethyl ether, POE (14) POP (7) dimethyl ether, POE (17) POP (4) dimethyl ether, POE (10) POP (10) dimethyl ether, POE (6) POP (14) dimethyl ether, POE (15) POP (5) dimethyl ether, POE (25) POP (25) dimethyl ether, POE (27) POP (14) dimethyl ether, POE (55) POP (28) dimethyl ether, POE (36) POP (41) dimethyl ether, POE (9) POB (2) dimethyl ether, POE (14) POB (7) dimethyl ether, POE (10) POP (10) diethyl ether, POE (10) POP (10) dipropyl ether, and POE (10) POP (10) dibutyl ether.

Examples of the water-soluble polymer include a plant-based polymer such as arabic rubber, carrageenan, pectin, agar, quince seed (marmelo), starch, or algae colloid (brown alga extract), a microbial polymer such as dextran or pullulan, an animal-based polymer such as collagen, casein, or gelatin, a starch-based polymer such as carboxymethyl starch or methylhydroxypropyl starch, an alginate-based polymer such as sodium alginate, a vinyl-based polymer such as sodium glutamate and a carboxyvinyl polymer (CARBOPOL or the like), a polyoxyethylene-based polymer, a polyoxyethylene polyoxypropylene copolymer-based polymer, an acrylic polymer such as sodium polyacrylate and polyacrylamide, an inorganic water-soluble polymer such as bentonite, aluminum magnesium silicate, or laponite.

Examples of the hydrophilic powder include an inorganic powder (for example, talc, kaolin, sericite, white mica, synthetic mica, golden mica, red mica, black mica, lithia mica, vermiculite, magnesium carbonate, calcium carbonate, diatomaceous earth, magnesium silicate, calcium silicate, aluminum silicate, barium silicate, strontium silicate, a metal tungstate, silica, hydroxyapatite, zeolite, boron nitride, or ceramic powder); an organic powder (for example, polyamide resin powder (nylon powder), polyethylene powder, polymethyl methacrylate powder, polystyrene powder, styrene/acrylic acid copolymer resin powder, benzoguanamine resin powder, polytetrafluoroethylene powder, or cellulose powder); an inorganic white pigment (for example, zinc oxide); an inorganic red pigment (for example, iron titanate); an inorganic purple pigment (for example, mango violet or cobalt violet); an inorganic green pigment (for example, chromium oxide, chromium hydroxide, or cobalt titanate); an inorganic blue pigment (for example, ultramarine blue, or iron blue); a pearl pigment (for example, titanium oxide coated mica, titanium oxide coated bismuth oxychloride, titanium oxide coated talc, colored titanium oxide coated mica, bismuth oxychloride, or argentine); a metal powder pigment (for example, aluminum powder or copper powder); an organic pigment such as zirconium, barium, or aluminum lake (for example, an organic pigment such as Red 201, Red 202, Red 204, Red 205, Red 220, Red 226, Red 228, Red 405, Orange 203, Orange 204, Yellow 205, Yellow 401, and Blue 404, Red 3, Red 104, Red 106, Red 227, Red 230, Red 401, Red 505, Orange 205, Yellow 4, Yellow 5, Yellow 202, Yellow 203, Green 3, and Blue 1; and a natural pigment (for example, chlorophyll or β-carotene).

### (c) Oily component

In the present invention, as an oily component (c), an oil usually used in a cosmetic product can be blended in a range that does not impair the effects of the present invention.

Examples of the oil that can be blended in the present invention include a hydrocarbon, an ester oil, a vegetable fat/oil, an animal fat/oil, a higher alcohol, a higher fatty acid, and a silicone oil.

Examples of the hydrocarbon include liquid paraffin, paraffin, squalane, squalene, ozokerite, pristine, celesin, Vaseline, and microcrystalline wax.

Examples of the ester oil include isopropyl myristate, cetyl octanoate, octyldodecyl myristate, isopropyl palmitate, butyl stearate, hexyl laurate, myristyl myristate, decyl oleate, hexyldecyl dimethyloctanoate, cetyl lactate, myristyl lactate, lanolin acetate, isocetyl stearate, isocetyl isostearate, cholesteryl 12-hydroxystearate, ethylene glycol di2-ethylhexanoate, dipentaerythritol fatty acid ester, N-alkyl glycol monoisostearate, neopentyl glycol dicaprate, diisostearyl malate, glycerin di2-heptylundecanoate, trimethylolpropane tri2-ethylhexanoate, trimethylolpropane triisostearate, glycerin trioctanoate, glycerin triisopalmitate, cetyl 2-ethylhexanoate, pentaerythritol tetra2-ethylhexanoate, glycerin tri2-ethylhexanoate, 2-ethylhexyl palmitate, glycerin trimyristate, glyceride tri2-heptylundecanoate, methyl castorate, oleyl oleate, acetoglyceride, 2-heptylundecyl palmitate, diisobutyl adipate, N-lauroyl-L-glutamic acid-2-octyldodecyl ester, di-2-heptylundecyl adipate, ethyl laurate, di-2-ethylhexyl sebacate, 2-hexyldecyl myristate, 2-hexyldecyl palmitate, di2-hexyldecyl adipate, diisopropyl sebacate, triethyl citrate, and diethylhexyl succinate.

Examples of the vegetable oil/fat include avocado oil, camellia oil, macadamia nut oil, corn oil, olive oil, rapeseed oil, sesame oil, castor oil, peanut oil, almond oil, soybean oil, tea seed oil, jojoba oil, and germ oil.

Examples of the animal fat/oil include turtle oil, egg yolk oil, and mink oil.

Examples of the higher alcohol include oleyl alcohol, stearyl alcohol, isostearyl alcohol, behenyl alcohol, octyldodecanol, decyltetradecanol, jojoba alcohol, cetyl alcohol, and myristyl alcohol, and examples of the higher fatty acid include oleic acid, isostearic acid, linolic acid, linoleic acid, eicosapentaenoic acid, palmitic acid, stearic acid, and behenic acid.

Examples of the silicone oil include chain polysiloxane (for example, dimethylpolysiloxane, methylphenylpolysiloxane, diphenylpolysiloxane, or methyl hydrogen polysiloxane), a silicone resin forming a three-dimensional network structure, silicone rubber, and an acrylic silicone.

In addition to the above, a "water-holding oil" capable of absorbing (holding) a large amount of water can also be blended. The water-holding oil is an oil having the property of holding water, and in particular, an oil having a water-holding capacity of 100% or more, that is, one capable of holding water equal to or greater than its own weight is preferable.

Examples of the water-holding oil include dipentaerythrit hexaoxystearate, pentaerythrit tetra(behenate/benzoate/ethylhexanoate), phytosteryl macadamiate, di(phytosteryl, 2-octyldodecyl) N-lauroyl-L-glutamate, glycerin triisostearate, and macadamia seed oil polyglyceryl-6 esters behenate.

Of the oils, a volatile oil can be preferably used in the present invention. This is because the oil phase, which is a continuous phase, contains a volatile oil, which is expected to improve usability such as fit to the skin and glossiness. Here, the volatile oil refers to an oil having a boiling point of 260°C or less at normal pressure (low boiling point oil). Typical examples thereof include an isoparaffin-based hydrocarbon oil and a silicone oil.

Examples of the low boiling point isoparaffin-based hydrocarbon oil include isododecane and isohexadecane. Examples of the low boiling point silicone oil include cyclic dimethylpolysiloxane having 4 to 6 silicon atoms and chain dimethylpolysiloxane having 2 to 5 silicon atoms.

In the water-in-oil emulsified cosmetic according to the present invention, one or two or more of the oils can be used. The preferable amount of an oil blended in the water-in-oil emulsified cosmetic of the present invention is 10 to 60% by mass, and particularly preferably 15 to 50% by mass.

In addition, in the present invention, an ultraviolet absorber can also be blended as the component (c).

Examples of the ultraviolet absorbers include a benzoic acid-based ultraviolet absorber (for example, paraaminobenzoic acid (hereinafter referred to as PABA), PABA monoglycerin ester, N,N-dipropoxy PABA ethyl ester, N,N-diethoxy PABA ethyl ester, N,N-dimethyl PABA ethyl ester, or N,N-dimethyl PABA butyl ester); an anthranilic acid-based ultraviolet absorber (for example, homomenthyl-N-acetylanthranilate); a salicylic acid-based ultraviolet absorber (for example, amyl salicylate, menthyl salicylate, homomenthyl salicylate, octyl salicylate, phenyl salicylate, benzyl salicylate, or p-isopropanol phenyl salicylate; a cinnamic acid-based ultraviolet absorber (for example, octylmethoxycinnamate, ethyl-4-isopropylcinnamate, methyl-2,5-diisopropylcinnamate, ethyl-2,4-diisopropylcinnamate, methyl-2,4-diisopropylcinnamate, propyl-p-methoxycinnamate, isopropyl-p-methoxycinnamate, isoamyl-p-methoxycinnamate, octyl-p-methoxycinnamate (2-ethylhexyl-p-methoxycinnamate), 2-ethoxyethyl-p-methoxycinnamate, cyclohexyl-p-methoxycinnamate, ethyl-α-cyano-β-phenylcinnamate, 2-ethylhexyl-α-cyano-β-phenylcinnamate, or glyceryl mono-2-ethylhexanoyl-diparamethoxycinnamate); a benzophenone-based ultraviolet absorber (for example, 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone, 2-hydroxy-4-methoxy-4'-methylbenzophenone, 2-hydroxy-4-methoxybenzophenone-5-sulfonate, 4-phenylbenzophenone, 2-ethylhexyl-4'-phenyl-benzophenone-2-carboxylate, 2-hydroxy-4-n-octoxybenzophenone, or 4-hydroxy-3-carboxybenzophenone); 3-(4'-methylbenzylidene)-d,1-camphor, 3-benzylidene-d,1-camphor; 2-phenyl-5-methylbenzoxazole; 2,2'-hydroxy-5-methylphenylbenzotriazole; 2-(2'-hydroxy-5'-t-octylphenyl)benzotriazole; 2-(2'-hydroxy-5'-methylphenylbenzotriazole; dibenzalazine; dianisoylmethane; 4-methoxy-4'-t-butyldibenzoylmethane; 5-(3,3-dimethyl-2-norbornylidene)-3-pentan-2-one, dimorpholinopyridazino; 2-ethylhexyl-2-cyano-3,3-diphenylacrylate; and 2,4-bis-{[4-(2-ethylhexyloxy)-2-hydroxy]-phenyl}-6-(4-methoxyphenyl)-(1,3,5)-triazine.

In addition, in the present invention, an oil-soluble agent, a fragrance, or the like may be blended as the component (c). Examples of the oil-soluble agent include an oil-soluble vitamin such as vitamin A or a vitamin A derivative.

In the present invention, the amount of (c) the oily component blended is 60% by mass or less, preferably 50% by mass or less, and more preferably 40% by mass or less. If the amount of the component (c) blended exceeds 60% by mass, it is difficult to obtain a water-in-oil emulsified cosmetic having all the effects of the present invention.

### (d) Emulsifier

In the present invention, as an emulsifier (d), one that can be usually used for a cosmetic can be used.

As the emulsifier, one having an HLB of 7 or less can be used, and one having an HLB of 5 or less is particularly preferable. The HLB value can be calculated by the Kawakami formula represented by HLB = 7 + 11.7. log(MW / MO) (where MW represents the molecular weight of the hydrophilic group moiety and MO represents the molecular weight of the lipophilic group moiety).

Examples of an emulsifier that can be suitably used in the present invention include an organically modified viscous mineral, a silicone-based surfactant, and a polyhydric alcohol fatty acid ester-based surfactant.

Examples of the organically modified viscous mineral include dimethylalkylammonium hectorite, benzyldimethylstearylammonium hectorite, and distearyldimethylammonium chloride treated aluminum magnesium silicate.

Examples of the silicone-based surfactant include a poly(oxyethylene/oxypropylene)methylpolysiloxane copolymer, a polyoxyethylene/methylpolysiloxane copolymer, a silicone chain branched methylpolysiloxane copolymer, and an alkyl chain branched polyoxyethylene methylpolysiloxane copolymer, an alkyl chain/silicone chain branched polyoxyethylene methylpolysiloxane copolymer, crosslinked polyoxyethylene methylpolysiloxane, alkyl group-containing crosslinked polyoxyethylene methylpolysiloxane, branched polyglycerin-modified silicone, and alkyl group-branched polyglycerin-modified silicone.

Examples of the polyhydric alcohol fatty acid ester-based surfactant include glycerin fatty acid ester, polyglycerin fatty acid ester, polyoxyethylene glycerin fatty acid ester, sorbitan fatty acid ester, and polyoxyethylene sorbitan fatty acid ester.

The amount of the emulsifier (d) blended in the water-in-oil emulsified cosmetic according to the present invention is preferably 0.01 to 10% by mass, and more preferably 0.1 to 5% by mass, based on the total amount of the composition.

### (e) Hydrophobized powder

A hydrophobized powder(e) may further be blended into the water-in-oil emulsified cosmetic according to the present invention.

Examples of the hydrophobized powder include one obtained by using a silicone (including a silicone elastomer) such as methyl hydrogen polysiloxane and dimethylpolysiloxane, a dextrin fatty acid ester, a higher fatty acid, a higher alcohol, a fatty acid ester, a metal soap, an alkylphosphoric acid ether, a fluorine compound, a hydrocarbon such as squalane or paraffin, or the like to hydrophobize the surface of a powder by a wet method using a solvent, a gas phase method, a mechanochemical method, or the like, or one obtained by coating a powder with silica and then hydrophobizing the coated powder with an alkyl-modified silane coupling agent or the like.

Of these, as the hydrophobization, treatment with a silicone is preferable, and elastomer treatment can be particularly preferably used. Examples of a silicone elastomer that can be preferably used in the present invention include an elastomer produced by mixing and heating a silicone polymer having an amino group (particularly preferably, a side chain type amino-modified silicone) and a silicone polymer having a carboxyl group (see WO 2017/209077).

Examples of the powder to be hydrophobized include, but are not particularly limited to, an inorganic white pigment such as talc, kaolin, sericite, white mica, titanium oxide, or iron oxide, an inorganic red pigment such as iron oxide (colcothar) or iron titanate, an inorganic yellow pigment such as yellow iron oxide or ocher, an inorganic purple pigment such as mango violet or cobalt violet, an inorganic green pigment such as chromium oxide, chromium hydroxide, or cobalt titanate, an inorganic blue pigment such as ultramarine blue or iron blue, a pearl pigment such as titanium oxide coated mica, titanium oxide coated bismuth oxyoxide, bismuth oxychloride, titanium oxide coated talc, argentine, or colored titanium oxide coated mica, a metal powder pigment such as aluminum powder or copper powder, an inorganic powder such as synthetic mica, golden mica, red mica, black mica, lithia mica, vermiculite, magnesium carbonate, calcium carbonate, diatomaceous earth, magnesium silicate, calcium silicate, aluminum silicate, barium silicate, strontium silicate, a metal tungstate, α-iron oxide, hydrated iron oxide, silica, or hydroxyapatite, and an organic powder such as nylon powder, polyethylene powder, benzoguanamine powder, microcrystalline cellulose, or silicone powder. Other examples thereof include an ultraviolet protection powder such as fine particle titanium oxide and fine particle zinc oxide, and a composite powder in which an organic powder is coated with an inorganic powder.

In the present invention, by blending the hydrophobized powder together with the component (a), the effect of giving smoothness to the skin and the non-stickiness can be further improved. In particular, when a silicone elastomer is used as the hydrophobized powder, a more remarkable effect can be obtained.

The amount of the hydrophobized powder blended in the water-in-oil emulsified cosmetic according to the present invention is 0.1 to 20% by mass, and preferably 1 to 10% by mass, based on the cosmetic. If the amount of the hydrophobized powder blended is less than 0.1% by mass, the cosmetic may not feel smooth, and if the amount of the hydrophobized powder blended exceeds 20% by mass, the ease of spreading may be lost.

The water-in-oil emulsified cosmetic according to the present invention can be appropriately blended with other ingredients usually used in a cosmetic product in a range that does not impair the effects of the present invention, to produce a cosmetic.

The product form of the cosmetic according to the present invention is not particularly limited, and examples thereof include a makeup cosmetic such as a foundation, a makeup base, a blusher, an eye shadow, an eyeliner, an eyebrow, or a mascara, and a skin care cosmetic such as a cream, a milky lotion, a facial mask, or a sunscreen makeup.

The water-in-oil emulsified cosmetic according to the present invention can be prepared according to a conventional method. For example, the water-in-oil emulsified cosmetic may be produced by mixing and dissolving an oil phase component, adding the resultant to an aqueous phase component while stirring, and emulsifying the resulting mixture.

### EXAMPLES

Hereinafter, the present invention will be described in more detail with reference to Examples, but the scope of the present invention should not be limited by these Examples. In addition, the amount blended in the present Example is in mass% unless otherwise specified.

First, the method for synthesizing the molecular weight controlled sodium polyacrylate used in the Examples will be shown.

### <Production Example 1>

A molecular weight controlled sodium polyacrylate was synthesized using the RAFT polymerization method of living radical polymerization. 4,4'-azobis-(4-cyanovaleric acid) (V-501, manufactured by Wako Pure Chemical Industries, Ltd.) as a polymerization initiator, and dithiobenzoate 4-cyanopentanoate as a chain transfer agent (synthesized according to Non-Patent Literature 1, hereinafter abbreviated as CPD) were used.

Acrylic acid (2514 mg), methylenebisacrylamide (9.6 µg), and V-501 (0.17 mg) were dissolved in ion-exchanged water (9 ml), a methanol solution (1 ml) in which CPD (0.17 mg) was dissolved was added, and the polymerization reaction was performed at 60°C for 24 hours in an argon atmosphere. After the polymerization reaction, an aqueous sodium hydroxide solution was added to perform neutralization to a pH of about 6 to 7, followed by dialysis with purified water for 4 days and then freeze-drying to collect molecular weight controlled sodium polyacrylate-3 (1.99 g, yield of 79%). As a result of analysis using GPC, the weight average molecular weight was 3.26 million and the molecular weight distribution was 1.7.

The component marked with * in the table below is as follows. *1: Talc JA-68R (manufactured by Asada Milling Co., Ltd.) surface-treated with an elastomer composed of an aminoethylaminopropylmethylsiloxane/dimethylsiloxane copolymer and PCA dimethicone (corresponding to the elastomer treated talc of Test Example 3-1 of WO 2017/209077).

The water-in-oil emulsified cosmetics having the recipes shown in Tables 1 to 4 were prepared according to the following production method, and physical properties thereof were evaluated by the following methods. Tables 1 and 2 are cream recipes, Table 3 is a foundation recipe, and Table 4 is a foundation recipe. Furthermore, the following items (7) to (10) were subjected to actual use tests using a specialized panel. Results thereof are also shown in each table.

### <Production method>

The oil phase components other than the elastomer treated talc were mixed and heated to about 80°C to dissolve the components, and then the elastomer treated talc was added and mixed and dispersed. On the other hand, the aqueous phase components were mixed and heated to about 80°C. Parts of the aqueous phase components were gradually added while stirring parts of the oil phase components relatively strongly, emulsified, and then stirred and cooled to obtain a water-in-oil emulsified cosmetic.

### <Physical property evaluation and the like>

### • Hardness

The hardness was measured using a rheometer (manufactured by FUDO kougyou, Inc.: NRM-3002D; diameter of 11.3 mm, 3-mm penetration) in an atmosphere of 25°C. In the present invention, when the hardness was 5 or more, it was determined that the hardness is appropriate for a cream.

### • Viscosity

The viscosity (mPa·s) of each sample immediately after preparation was measured at 30°C using a rotary viscometer (BL type, 12 rpm, 1 minute).

### • Emulsified particle size

The size was observed using an optical microscope (manufactured by Olympus Corporation: BX60).

### • Formulation

In each table, when the desired dosage form of the cosmetic was obtained, A was given, and when the desired dosage form of the cosmetic was not obtained, C was given.

### <Actual use test>

10 specialized panelists were asked to apply the test composition to their faces, and asked to answer whether or not the test composition had effects on rich feeling, spreadability, skin moisturization, skin smoothness, and non-stickiness. The answer results were summarized according to the following criteria and are indicated in the table. S: 9 or more answered that the test composition was effective.
A: 7 or more and 8 or fewer answered that the test composition was effective.
B: 5 or more and 6 or fewer answered that the test composition was effective.
C: 4 or fewer answered that the test composition was effective.
In the present invention, S and A were considered passing, and B and C were considered failing.

**[Table 1]**

| | | | Comparative Example 1 | Example1 | Example 2 |
|---|---|---|---|---|---|
| | c | Dimethicone | 10 | 10 | 10 |
| | | Diphenylsiloxyphenyltrimethicone | 2 | 2 | 2 |
| | | Cetyl ethylhexanoate | 1 | 1 | 1 |
| | | Hydrogenated polydecene | 2 | 2 | 2 |
| | d | PEG-9 Polydimethylsiloxyethyl dimethicone | 0,5 | 0,5 | 0,5 |
| | | PEG/PPG-19/19 Dimethicone | 1 | 1 | 1 |
| | e | Elastomer treated talc | | | 3 |
| | a | Molecular weight controlled sodium polyacrylate (Production Example 1) | | 0,1 | 0,1 |
| **Recipe** | b | Ethanol | 10 | 10 | 10 |
| | | Glycerin | 6 | 6 | 6 |
| | | Dipropylene glycol | 6 | 6 | 6 |
| | | Sorbitol | 2,5 | 2,5 | 2,5 |
| | | PEG-150 | 0,5 | 0,5 | 0,5 |
| | | PEG/PPG-14/7 Dimethyl ether | 2 | 2 | 2 |
| | | Citric acid | 0,3 | 0,3 | 0,3 |
| | | Sodium citrate | 0,2 | 0,2 | 0,2 |
| | | Phenoxyethanol | 0,5 | 0,5 | 0,5 |
| | | EDTA-3Na · 2H₂O | 0,05 | 0,05 | 0,05 |
| | | Salt | 1 | 1 | 1 |
| | | lon-exchanged water | Balance | Balance | Balance |
| **Evaluation** | Amount of component (c) blended (% by mass) | | 15 | 15 | 15 |
| | Hardness | | 13 | 6 | 6 |
| | Emulsified particle size (mm) | | ∼ 2.5 | 1∼5 | 1∼5 |
| | Rich feeling | | A | A | A |
| | Spreadability | | C | S | S |
| | Skin moisturization | | A | S | A |
| | Skin smoothness | | A | A | S |
| | Non-stickiness | | B | A | S |

As shown in Table 1, the cream of Comparative Example 1 spread poorly and felt sticky, but the cream of Example 1 in which the molecular weight controlled sodium polyacrylate (a) (Production Example 1) was added as an aqueous phase component to the recipe spread very well, was excellent in non-stickiness, and was also quite excellent in the effect of giving moisturization to the skin. In addition, the cream was also excellent in the effect of giving a rich feeling and smoothness to the skin (Example 1).

Furthermore, the cream in which elastomer treated talc was added to the recipe of Example 1 (Example 2) was also quite excellent in the effect of giving smoothness to the skin and non-stickiness.

**[Table 2]**

| | | | Comparative Example 2 | Example 3 | Example 4 | Comparative Example 3 | Example 5 | Comparative Example 4 | Example 6 | Comparative Example 5 | Comparative Example 6 |
|---|---|---|---|---|---|---|---|---|---|---|---|
| | **c** | Hydrogenated polydecene | 5 | 5 | 5 | 15 | 15 | 20 | 20 | 4,5 | 4,5 |
| | | Isohexadecane | 5 | 5 | 5 | 18 | 18 | 24 | 24 | 5 | 5 |
| | | Isododecane | 6 | 6 | 6 | 6 | 6 | 6 | 6 | 5,95 | 5,95 |
| | | Cetyl ethylhexanoate | 5 | 5 | 5 | 5 | 5 | 5 | 5 | 5 | 5 |
| | | Dimethicone | 5 | 5 | 5 | 5 | 5 | 5 | 5 | 5 | 5 |
| | **d** | Distearyldimonium hectorite | 2 | 2 | 2 | 2 | 2 | 2 | 2 | 2 | 2 |
| | | PEG-9 Polydimethytsiloxyethyl dimethicone | 2 | 2 | 2 | 2 | 2 | 2 | 2 | 2 | 2 |
| | | PEG-8/PEG-8 Diisostearate | 1 | 1 | 1 | 1 | 1 | 1 | 1 | 1 | 1 |
| | **e** | Elastomer treated talc | | | 5 | | | | | | |
| **Recipe** | **a** | Molecular weight controlled sodium polyacrylate (Production Example 1) | | 0,1 | 0,1 | | 0,1 | | 0,1 | | 0,1 |
| | **b** | Glycerin | 12 | 12 | 12 | 12 | 12 | 12 | 12 | 12 | 12 |
| | | PEG-150 | 1 | 1 | 1 | 1 | 1 | 1 | 1 | 1 | 1 |
| | | Dipropylene glycol | 8 | 8 | 8 | 8 | 8 | 8 | 8 | 8 | 8 |
| | | EDTA-3Na · 2H₂O | 0,05 | 0,05 | 0,05 | 0,05 | 0,05 | 0,05 | 0,05 | 0,05 | 0,05 |
| | | Citric acid | 0,02 | 0,02 | 0,02 | 0,02 | 0,02 | 0,02 | 0,02 | 0,02 | 0,02 |
| | | Sodium citrate | 0,08 | 0,08 | 0,08 | 0,08 | 0,08 | 0,08 | 0,08 | 0,08 | 0,08 |
| | | Phenoxyethanol | 0,3 | 0,3 | 0,3 | 0,3 | 0.3 | 0,3 | 0,3 | 0,3 | 0,3 |
| | | Ethylparaben | 0,1 | 0,1 | 0,1 | 0,1 | 0,1 | 01 | 0,1 | 0,1 | 0,1 |
| | | Methylparaben | 0,2 | 02 | 0,2 | 0.2 | 0,2 | 0,2 | 0,2 | 0,2 | 02 |
| | | lon-exchanged water | Balance | Balance | Balance | Balance | Balance | Balance | Balance | Balance | Balance |
| **Evaluation** | Amount of component (c) blended (% by mass) | | 26 | 26 | 26 | 49 | 49 | 60 | 60 | 68 | 68 |
| | Hardness | | 20 | 8 | 9 | 5 | 5 | 4 | 7 | - | 3 |
| | Emulsified particle size (mm) | | ∼2.5 | ∼5 | ∼5 | ∼2.5 | ∼5 | ∼2.5 | ∼5 | ∼2.5 | ∼5 |
| | Formulation | | A | A | A | A | A | A | A | C | A |
| | Rich feeling | | A | A | S | B | S | B | S | - | A |
| | Spread ability | | C | S | A | C | S | C | A | - | B |
| | Skin moisturization | | A | A | S | A | A | A | A | - | C |
| | Skin smoothness | | A | S | S | A | A | A | A | - | B |
| | Non-stickiness | | B | A | S | B | A | B | A | - | C |

In addition, as shown in Table 2, the cream of Comparative Example 2 spread poorly and felt sticky, but the cream of Example 3 in which the molecular weight controlled sodium polyacrylate (a) (Production Example 1) was added as an aqueous phase component to the recipe spread very well, was excellent in non-stickiness, and was also quite excellent in the effect of giving moisturization to the skin. In addition, the cream was also excellent in the effect of giving a rich feeling and smoothness to the skin (Example 3).

Furthermore, the cream (Example 4) in which elastomer treated talc was added to the recipe of Example 3 was also quite excellent in the effect of giving smoothness to the skin and non-stickiness.

The above is a comparison between creams in which the amount of the component (c) blended is relatively small (the amount blended is 15% by mass or 26% by mass), but even in the creams in which the amount blended was 49% by mass (Comparative Example 3 and Example 5) and the creams in which the amount blended was 60% by mass (Comparative Example 4 and Example 6), by adding the molecular weight controlled sodium polyacrylate (a) (Production Example 1), the rich feeling, the spreadability, and the non-stickiness are remarkably improved (Comparative Example 3 vs Example 5) and 60% by weight (Comparative Example 4 vs Example 6).

Furthermore, when the amount of the oil components blended was increased until the amount of the component (c) blended became 68% by mass, a dosage form called a cream was not obtained (Comparative Example 5), but by adding molecular weight controlled sodium polyacrylate (a) (Production Example 1) to the recipe, a cream was obtained (Comparative Example 6). However, with the cream of Comparative Example 6, none of the spreadability, skin moisturization, skin smoothness, and non-stickiness was obtained.

Therefore, it was indicated that when a molecular weight controlled polyacrylate is blended to the internal aqueous phase of a water-in-oil emulsified cosmetic in which the amount of the component (c) blended is 60% by mass or less, the spread is very good, the stickiness decreases, and the rich feeling increases, and the effect of giving moisturization and smoothness to the skin is far superior.

Next, the difference in effect from a polyacrylate whose molecular weight was not controlled was examined. As the polyacrylate, commercially available polyacrylic acids (manufactured by Sigma-Aldrich) having a weight average molecular weight of 750,000 and 4 million, respectively, neutralized with sodium hydroxide in the same manner as in Production Example 1 of the present application were used.

**[Table 3]**

| | | | Comparative Example 2 | Comparative Example7 | Comparative Example 8 | Example 3 |
|---|---|---|---|---|---|---|
| | c | Hydrogenated polydecene | 5 | 5 | 5 | 5 |
| | | Isohexadecane | 5 | 5 | 5 | 5 |
| | | Isododecane | 6 | 6 | 6 | 6 |
| | | Cetyl ethylhexanoate | 5 | 5 | 5 | 5 |
| | | Dimethicone | 5 | 5 | 5 | 5 |
| | | Dipropylene glycol | 8 | 8 | 8 | 8 |
| | d | Distearyldimonium hectorite | 2 | 2 | 2 | 2 |
| | | PEG-9 Polydimethylsiloxyethyl dimethicone | 2 | 2 | 2 | 2 |
| | | PEG-8/PEG-8Diisostearate | 1 | 1 | 1 | 1 |
| **Recipe** | a | Molecular weight controlled sodium polyacrylate (Production Example 1) | | | | 0,1 |
| | b | Sodium polyacrylate(750,000) | | 0.1 | | |
| | | Sodium polyacrylate(4 million) | | | 0,1 | |
| | | Glycerin | 12 | 12 | 12 | 12 |
| | | PEG-150 | 1 | 1 | 1 | 1 |
| | | EDTA-3Na · 2H₂O | 0,05 | 0,05 | 0,05 | 0,05 |
| | | Citric acid | 0,02 | 0,02 | 0,02 | 0,02 |
| | | Sodium citrate | 0,08 | 0,08 | 0,08 | 0,08 |
| | | Phenoxyethanol | 0,3 | 0,3 | 0,3 | 0,3 |
| | | Ethylparaben | 0,1 | 0,1 | 0.1 | 0,1 |
| | | Methylparaben | 0,2 | 0,2 | 0,2 | 0,2 |
| | | Ion-exchanged water | Balance | Balance | Balance | Balance |
| **Evaluation** | Amount of component (c) blended (% by mass) | | 26 | 26 | 26 | 26 |
| | Hardness | | 20 | 15 | 15 | 8 |
| | Emulsified particle size (mm) | | ∼2.5 | ∼5 | ∼5 | ∼5 |
| | Formulation | | A | A | A | A |
| | Rich feeling | | A | A | A | A |
| | Spreadability | | C | C | C | S |
| | Skin moisturization | | A | A | A | A |
| | Skin smoothness | | A | A | A | S |
| | Non-stickiness | | B | A | A | A |

As shown in Table 3, when a polyacrylic acid whose molecular weight was not controlled was added to the recipe of Comparative Example 2, the non-stickiness was improved, but the spreadability and the effect of giving moisturization and smoothness to the skin remained unchanged (Comparative Examples 7 and 8).

Therefore, it was indicated that the improvement in feeling on use, especially the remarkable improvement in the spreadability and the effect of giving moisturization and smoothness to the skin, is due to the controlled molecular weight of the polyacrylate added to the aqueous phase.

**[Table 4]**

| | | | Comparative Example 9 | Comparative Example 7 | Example 8 |
|---|---|---|---|---|---|
| | **c** | Dimethicone Cyclopenta siloxane Ethylhexyl methoxycinnamate Diethylhexyl Succinate | 15 | 15 | 15 |
| | | | 8 | 8 | 8 |
| | | | 2 | 2 | 2 |
| | | | 2 | 2 | 2 |
| | | Diphenylsiloxyphenyl trimethicone | 3 | 3 | 3 |
| | | (Dimethicone/Phenytvinyl dimethicone) copolymer | 2 | 2 | 2 |
| | **d** | PEG-10 Dimethicone Bis-Butyldimethicone Polyglyceryl-3 Distearyldimonium hectorite | 5 | 5 | 5 |
| | | | 2 | 2 | 2 |
| | | | 2 | 2 | 2 |
| | **e** | Alkylation modified silicone treated red iron oxide | 1 | 1 | 1 |
| **Recipe** | | Alkylation modified silicone treated yellow iron oxide | 2,5 | 2,5 | 2,5 |
| | | Alkylation modified silicone treated black iron oxide | 0,1 | 0,1 | 0,1 |
| | | Elastomer treated talc | | | 5 |
| | **a** | Molecular weight controlled sodium polyacrylate (Production Example 1) | | 0,1 | 0,1 |
| | **b** | Titanium dioxide | 2 | 2 | 2 |
| | | EDTA-3Na · 2H₂O | 0,02 | 0,02 | 0,02 |
| | | Citric acid | 0,05 | 0,05 | 0,05 |
| | | Sodium citrate | 0,05 | 0,05 | 0,05 |
| | | Glycerin | 5 | 5 | 5 |
| | | Dipropylene glycol | 7 | 7 | 7 |
| | | Phenoxyethanol | 0,5 | 0,5 | 0,5 |
| | | lon-exchanged water | Balance | Balance | Balance |
| **Evalua tion** | Amount of component (c) blended (% by mass) | | 32 | 32 | 32 |
| | Hardness | | 33200 | 34650 | 39200 |
| | Emulsified particle size (mm) | | ∼1 | ∼1 | ∼1 |
| | Rich feeling | | A | S | S |
| | Spreadability | | C | S | A |
| | Skin moisturization | | A | S | S |
| | Skin smoothness | | B | A | S |
| | Non-stickiness | | A | S | S |

As shown in Table 4, the foundation(Example 7) in which the molecular weight-controlled sodium polyacrylate (a) (Production Example 1) was added to the recipe of Comparative Example 9 was superior in rich feeling, spreadability, the effect of giving moisturization and smoothness to the skin, and non-stickiness to the foundation having the recipe of Comparative Example 9. The foundation to which elastomer treated talc was further added (Example 8) was also superior in the effect of giving smoothness to the skin.

From the above results, it was indicated that when a molecular weight controlled polyacrylate is blended into a water-in-oil emulsified cosmetic in which the amount of an oily component (c) blended is 60% by mass or less, the spreading is very good, the sticky feeling decreases, the rich feeling increases, and the effect of giving moisturization and smoothness to the skin is far superior.

The following is recipe examples of the water-in-oil emulsified cosmetic of the present invention. It goes without saying that the present invention is not limited by these recipe examples. In addition, all the amounts blended is expressed in % by mass based on the total amount of the cosmetic.

### Formulation example 1: Body cream

| Component | Blending amount (by mass) |
|---|---|
| Dimethylpolisiloxane | 3 |
| Decamethylcyclopentasiloxane | 13 |
| Dodecamethylcyclohexasiloxane | 12 |
| Polyoxyethylene/methylpolysiloxane copolymer | 1 |
| Ethanol | 2 |
| Isopropanol | 1 |
| Glycerin | 3 |
| Dipropylene glycol | 5 |
| Polyethylene glycol 6000 | 5 |
| Sodium hexametaphosphate | 0.05 |
| Tocopherol acetate | 0.1 |
| Caffeine | 0.1 |
| Fennel extract | 0.1 |
| Hamamelier extract | 0.1 |
| Carrot extract | 0.1 |
| L-menthol | q.s. |
| Paraoxybenzonic acid ester | q.s. |
| Torisodium edetate | 0.05 |
| Dimorpholinopyridazinone | 0.01 |
| Methyl bis(trimethylsiloxy)silylisopentyl trimethoxycinnamate | 0.1 |
| Yellow iron oxide | q.s. |
| Cobalt titanate | q.s. |
| Quaternary ammonium compounds | 1.5 |
| Molecular weight controlled sodium polyacrylate (Production Example 1) | 0.2 |
| Polyvinyl alcohol | 0.1 |
| Hydroxyethyl cellulose | 0.1 |
| Trimethylsiloxysilicic acid | 2 |
| Fragrance | q.s. |
| Purified water | Balance |
| Total | 100.00 |

### Formulation example 2 : Sunscreen cream

| Component | Blending amount (by mass) |
|---|---|
| Decamethylcyclopentasiloxane | 20 |
| Trimethylsiloxysilicic acid | 1 |
| Polyoxyethylene/methylpolysiloxane copolymer | 2 |
| Dipropylene glycol | 4 |
| Squalane | 5 |
| Silicone coated fine particles titanium oxide | 10 |
| Hydrophobicized talc | 6 |
| Paraben | q.s. |
| Phenoxyethanol | q.s. |
| Molecular weight controlled sodium polyacrylate (Production Example 1) | 0..05 |
| Torisodium edetate | 0.02 |
| 4-tert-butyl-4'-Methoxydibenzoylmethane | 0.1 |
| 2-Ethylhexyl paratoxy dermal acid | 7 |
| Glyceryl mono-2-ethylhexanoate diparamethoxycinnamate | 0.5 |
| Spherial polyethylene powder | 5 |
| Quaternary ammonium compounds | 1 |
| Fragrance | q.s. |
| Purified water | Balance |
| Total | 100.00 |

### Formulation example 3 : Sunscreen cream

| Component | Blending amount (by mass) |
|---|---|
| Dimethylpolisiloxane | 5 |
| Decamethylcyclopentasiloxane | 20 |
| Trimethylsiloxysilicic acid | 3 |
| Dimethylpolisiloxane | 5 |
| Polyoxyethylene/methylpolysiloxane copolymer | 3 |
| Dipropylene glycol | 3 |
| 2-Hexydecyl isostearate | 1 |
| Silicone coated fine particles zinc oxide | 10 |
| Talc | 1 |
| Silicone coated fine particles titanium oxide | 7 |
| Paraben | q.s. |
| Phenoxy ethanol | q.s. |
| Molecular weight controlled sodium polyacrylate (Production Example 1) | 0.005 |
| Torisodium edetate | 0.2 |
| Quaternary ammonium compounds | 1 |
| Polymethylmethacrylic acid copolymer spherical powder | 3 |
| Fragrance | q.s. |
| Purified water | Balance |
| Total | 100.00 |

### Formulation example 4: Sunscreen cream

| Component | Blending amount (by mass) |
|---|---|
| Decamethylcyclopentasiloxane | 20 |
| Ethanol | 5 |
| Isostearyl alcohol | 2 |
| Dipropylene glycol | 3 |
| Isostearic acid | 2 |
| Gryceryl tri2-ethylhexanoate | 5 |
| 2-Hexydecyl isostearate | 2 |
| Dextrin fatty acid ester coating fine particle titanium oxide | 2 |
| Sodium chloride | 2 |
| Torisodium edetate | q.s. |
| Uvinal®T-150 (product by BASF C.O.) | 1 |
| 4-tert-butyl-4'-Methoxydibenzoylmethane | 1 |
| 2-ethyhexyl paratoxy dermal acid | 7.5 |
| Sodium carboxymethyl cellulose | 0.5 |
| Molecular weight controlled sodium polyacrylate | |
| (Production Example 1) | 1 |
| Ethyl cellulose | 1 |
| Spherical acrylic resin powder | 5 |
| Fragrance | q.s. |
| Purified water | Balance |
| Total | 100.00 |

### Formulation example 5 : Cream

| Component | Blending amount (by mass) |
|---|---|
| Decamethylcyclopentasiloxane | 20 |
| α-Olefin oligomer | 10 |
| Vaseline | 1 |
| Microcrystalline wax | 3 |
| Decamethylcyclopentasiloxane | 5 |
| Glycerin | 10 |
| Dipropylene glycol | 2 |
| 1,3-Butylene glycol | 2 |
| Erythritol | 2 |
| Squalane | 1 |
| glycerin fatty acid ester eicosanedioic acid condensate | 0.1 |
| Isostearic acid | 1 |
| 2-Hexydecyl isostearate | 5 |
| Sodium chloride | 0.5 |
| Sodium hexametaphosphate | 0.05 |
| *Glycyrrhiza glabra* | 0.05 |
| Yeast extract | 0.1 |
| L-Ascorbyl phosphate | 2 |
| Tocophero acetate | 0.5 |
| Thiotaurin | 0.1 |
| DL-Pyrrolidone sodium carboxylate | 1 |
| Turmeric extract | 0.1 |
| Torisodium edetate | 0.1 |
| Quaternary ammonium compounds | 2 |
| Sodium carboxymethyl cellulose | 0.1 |
| Molecular weight controlled sodium polyacrylate (Production Example 1) | 0.5 |
| Paraben | q.s. |
| Fragrance | q.s. |
| Purified water | Balance |
| Total | 100.00 |

### Formulation example 6: Cream

| Component | Blending amount (by mass) |
|---|---|
| Mineral oil | 2 |
| Cetyl PEG/PPG-10/ Dimethicone | 2 |
| Lauryl PEG-9 polydimethylsiloxyethyl dimethicone | 2 |
| Cyclomethicone | 8 |
| Trimethylsiloxysilicic acid | 2 |
| (Alkyl alkylate/ dimethicone) copolymer | 1 |
| Ethanol | 5 |
| Glycerin | 1 |
| 1,3-Butylene glycol | 8 |
| Triethylhexanoin | 4 |
| Dipivalic acid PPG-3 | 1 |
| Isopropyl myristate | 5 |
| Isopropyl sebacate | 5 |
| Hydrophobicized titanium | 15 |
| Phenoxyethanol | q.s. |
| Disodium edetate | q.s. |
| t-Butyl methoxydibenzoyl methane | 2 |
| Octocrylene | 8 |
| Quaternary ammonium compounds | 1 |
| Polymethylmethacrylate | 5 |
| Molecular weight controlled sodium polyacrylate (Production Example 1) | 0.3 |
| Toranexamic acid | 2 |
| Fragrance | q.s. |
| Purified water | Balance |
| Total | 100.00 |

### Formulation example 7: Cream

| Component | Blending amount (by mass) |
|---|---|
| Mineral oil | 2 |
| PEG-9 Polydimethylsiloxyethyl dimethicone | 1 |
| Dimethicone | 12 |
| PEG-10 dimethicone crosspolymer | 3 |
| Ethanol | 5 |
| Glycerin | 5 |
| Dipropylene glycol | 3 |
| 1,3-Butylene glycol | 2 |
| PEG-150 | 1 |
| Molecular weight controlled sodium polyacrylate (Production Example 1) | 2 |
| Polymethylsilses quioxane | 1 |
| Citric acid | 0.15 |
| Sodium citrate | 0.05 |
| Salt | 1 |
| Toranexamic acid | 2 |
| Phenoxy ethanol | 0.35 |
| Disodium edetate | 0.1 |
| Cellulose gum | 0.25 |
| Fragrance | q.s. |
| Purified water | Balance |
| Total | 100.00 |

### Formulation example 8 : Cream

| Component | Blending amount (by mass) |
|---|---|
| Decamethylcyclopentasiloxane | 15 |
| Trimethylsiloxysilicic acid | 5 |
| Polyoxyethylene/methylpolysiloxane copolymer | 5 |
| Glycerin | 5 |
| 1,3-Butylene glycol | 5 |
| Maltitol liquid | 2 |
| Molecular weight controlled sodium polyacrylate (Production Example 1) | 0.1 |
| Macademia nuts oil | 2 |
| Squalane | 2 |
| Cholesteryl hydroxysrearate | 0.5 |
| 2-Hexydecyl isostearate | 2 |
| Distearyldimethyl ammonium chloride | 0.2 |
| L-ascorbic acid sulfate disodium | 0.1 |
| Potassium α-tocopherol 2-L-ascorbate phosphate diester | 0.1 |
| Tocophero acetate | 0.05 |
| Fish collagen | 0.4 |
| Sodium chondroitin sulfate | 0.01 |
| Sodium hyaluronate | 0.1 |
| Torisodium edetate | 0.05 |
| Glyceryl mono-2-ethylhexanoate diparamethoxycinnamate | 0.05 |
| Aluminum magnesium silicate | 0.3 |
| Paraben | q.s. |
| Purified water | Balance |
| Total | 100.00 |

### Formulation example 9 : Foundation

| Component | Blending amount (by mass) |
|---|---|
| Dimethylpolisiloxane | 3 |
| Decamethylcyclopentasiloxane | 10 |
| Polyoxyethylene/methylpolysiloxane copolymer | 3 |
| Dodecamethylcyclohexasiloxane | 5 |
| Glycerin | 4 |
| 1,3-Butylene glycol | 5 |
| Molecular weight controlled sodium polyacrylate (Production Example 1) | 0.5 |
| Palmitic acid | 0.5 |
| Distearyldimethyl ammonium chloride | 0.2 |
| Metal soap treated talc | 2 |
| Crosslinked silicone powder (TREFIL E--506) | 0.1 |
| Red oxide coated mica titanium | 0.5 |
| N-lauroyl-L-lisin | 2 |
| Monosodium L-glutamate | 2 |
| Tocopherol acetate | 0.1 |
| δ-Tocopherol | 0.1 |
| Paraoxybenzonic acid ester | q.s. |
| Phenoxyethanol | 0.2 |
| Spherical nylon powder | 1 |
| Spherical alkyl polyacrylate powder | 3 |
| Melilot extract | 2 |
| Dextrin fatty acid treated talc | 3 |
| Dextrin fatty acid treated titanium talc | 15 |
| Dextrin fatty acid treated yellow iron oxide | 3 |
| Dextrin fatty acid treated black iron oxide | 0.5 |
| Purified water | Balance |
| Total | 100.00 |

### Formulation example 10 : Foundation

| Component | Blending amount (by mass) |
|---|---|
| Dimethylpolisiloxane | 15 |
| Decamethylcyclopentasiloxane | 20 |
| Polyoxyethylene/methylpolysiloxane copolymer | 5 |
| High molecular weight amino-modified silicone | 0.1 |
| Glycerin | 5 |
| 1,3-Butylene glycol | 10 |
| Molecular weight controlled sodium polyacrylate (Production Example 1) | 0.1 |
| Palmitic acid | 0.5 |
| Macademia nut oil fatty acid treated talc | 0.1 |
| Distearyldimethyl ammonium chloride | 0.2 |
| Alkylation modified silicone resin coated yellow iron oxide | 2 |
| Alkylation modified silicone resin coated red oxide | 1 |
| Alkylation modified silicone resin coated black iron oxide | 0.3 |
| Alkylation modified silicone resin coated titanium oxide | 10 |
| Alkylation modified silicone resin coated talc | 1.5 |
| Silicone coated fusiform titanium oxide | 3 |
| DL- α tocopherol acetate | 0.1 |
| Paraoxybenzonic acid ester | q.s. |
| Methyl bis(trimethylsiloxy)silylisopentyl trimethoxycinnamate | 0.1 |
| Quaternary ammonium compounds | 1.5 |
| Spherical nylon powder | 1 |
| Fragrance | q.s. |
| Purified water | Balance |
| Total | 100.00 |

### Formulation example 11 : Foundation

| Component | Blending amount (by mass) |
|---|---|
| Dimethylpolisiloxane | 3 |
| Decamethylcyclopentasiloxane | 15 |
| Polyoxyethylene/methylpolysiloxane copolymer | 3 |
| Glycerin | 3 |
| 1,3-Butylene glycol | 5 |
| Molecular weight controlled sodium polyacrylate (Production Example 1) | 0.1 |
| Palmitic acid | 0.5 |
| Distearyldimethyl ammonium chloride | 0.2 |
| Glycerol-modified silicone resin-coated sericite | 0.5 |
| Dextrin fatty acid coated yellow iron oxide coated mica titanium | 0.5 |
| Dextrin fatty acid coated titanium dioxide | 2 |
| Dextrin fatty acid coated iron oxide/ titanium oxide sintered product (PK) | 12 |
| Dextrin fatty acid coated talc | 10 |
| Methyl hydrogen polysiloxane treated titanium oxide coated sericite | 0.5 |
| Boron nitride | 0.5 |
| Titanium oxide fine particle | 0.5 |
| Red oxide coated mica titanium | 0.5 |
| Phytosterol | 0.1 |
| Monosodium L-glutamate | 1.5 |
| Ascorbyl dipalmitate | 0.1 |
| DL- α tocopherol acetate | 0.1 |
| Acetylated sodium hyaluronate | 0.1 |
| Paraoxybenzonic acid ester | q.s. |
| Phenoxyethanol | q.s. |
| Red oxide coated mica titanium | 0.5 |
| Dextrin fatty acid coated yellow iron oxide | 2 |
| Dextrin fatty acid coated black iron oxide | 0.2 |
| Spherical nylon powder | 1 |
| Purified water | Balance |
| Total | 100.00 |

### Formulation example 12: Double layered type foundation

| Component | Blending amount (by mass) |
|---|---|
| Decamethylcyclopentasiloxane | 10 |
| Dodecamethylcyclohexasiloxane | 20 |
| Trimethylsiloxysilicic acid | 1 |
| Poly (oxyethylene/ oxypropylene) methylpolysiloxane copolymer | 3 |
| Ethanol | 10 |
| Molecular weight controlled sodium polyacrylate (Production Example 1) | 0.3 |
| Isostearic acid | 0.5 |
| Alkylation modified silicone resin coated titanium oxide | 10 |
| Dextrin palmitate coated titanium oxide | 5 |
| Dextrin palmitate coated talc | 5 |
| Needle shaped fine particles titanium oxide | 1 |
| Spherical silicic acid anhydride | 5 |
| Silicate anhydrous coated mica | q.s. |
| Sodium citrate | q.s. |
| N-lauroyl-L-lisin | 0.5 |
| DL- α tocopherol acetate | 0.1 |
| D- δ-tocopherol | 0.1 |
| Sophora angustifolia root extract | 1 |
| Dextrin palmitate coated red oxide | q.s. |
| Dextrin palmitate coated yellow iron oxide | q.s. |
| Dextrin palmitate coated black iron oxide | q.s. |
| Melilot extract | 2 |
| Purified water | Balance |
| Total | 100.00 |

### Formulation example 13 : Cream type foundation

| Component | Blending amount (by mass) |
|---|---|
| Dimethylpolisiloxane 6mPas | 5 |
| Decamethylcyclopentasiloxane | 25 |
| Polyoxyethylene/methylpolysiloxane copolymer | 3 |
| Glycerin | 1 |
| 1,3-Butylene glycol | 5 |
| Xylit | 0.5 |
| Molecular weight controlled sodium polyacrylate (Production Example 1) | 1 |
| Isostearic acid | 0.5 |
| Alkylation modified silicone resin coated silicic anhydride | 2 |
| Talc | 0.5 |
| Aluminum stearate | 1 |
| Red iron oxide coated mica titanium | 0.1 |
| Sodium hexametaphosphate | 0.05 |
| Dipotassium glycyrrhizinate | 0.1 |
| L-serine | 0.1 |
| Hyrericum extract | 0.1 |
| DL- α tocopherol acetate | 0.2 |
| Thiotaurin | 0.1 |
| *Rosa roxburghii* extract | 0.1 |
| Peony extract | 0.1 |
| Acetylated sodium hyaluronate | 0.1 |
| Saxifraga extract | 0.1 |
| Paraoxybenzonic acid ester | q.s. |
| Phenoxyethanol | q.s. |
| Dextrin palmitate coated yellow iron oxide | 0.1 |
| Quaternary ammonium compounds | 1 |
| Trimethylsiloxysilicic acid | 1.5 |
| Spherical silicic acid anhydride | 5 |
| Spherical polyethylene powder | 5 |
| Fragrance | q.s. |
| Purified water | Balance |
| Total | 100.00 |

### Formulation example 14 : Makeup base

| Component | Blending amount (by mass) |
|---|---|
| Dimethylpolisiloxane 6mPas | 5 |
| Decamethylcyclopentasiloxane | 30 |
| Polyoxyethylene/methylpolysiloxane copolymer | 3 |
| Dodecamethylcyclohexasiloxane | 1 |
| Glycerin | 5 |
| Dipropylene glycol | 5 |
| Molecular weight controlled sodium polyacrylate (Production Example 1) | 0.02 |
| Sage oil | 0.1 |
| Talc | 0.1 |
| Mica titanium | 0.1 |
| Crosslinked silicone powder (TREFIL E--506) | 3 |
| Polymethylsilsesquioxane powder | 10 |
| Tocopherol acetate | 0.1 |
| δ-tocopherol | 0.1 |
| Thiotaurin | 0.1 |
| Peppermint extract | 0.1 |
| Paraoxybenzonic acid ester | q.s. |
| Phenoxyethanol | q.s. |
| Torisodium edetate | q.s. |
| Colored pigments | q.s. |
| Dimethylstearylammonium hectorite | 1.5 |
| Purified water | Balance |
| Total | 100.00 |

### Formulation example 15 :Concealer

| Component | Blending amount (by mass) |
|---|---|
| Dimethylpolisiloxane | 2 |
| Decamethylcyclopentasiloxane | 15 |
| Polyoxyethylene/methylpolysiloxane copolymer | 3 |
| Glycerin | 5 |
| 1,3-Butylene glycol | 6 |
| D-mannitol | 1 |
| Molecular weight controlled sodium polyacrylate (Production Example 1) | 0.5 |
| Squalane | 0.5 |
| Dextrin fatty acid coated titanium oxide | 10 |
| Dextrin fatty acid coated talc | 10 |
| Spherical polymethylsilsesxion powder | 0.1 |
| Red iron oxide coated mica titanium | 0.5 |
| L-monosodium glutamate | 2 |
| Wild rose extract | 0.1 |
| Phenoxyethanol | q.s. |
| Red iron oxide coated mica titanium | 0.5 |
| Dextrin fatty acid coated yellow iron oxide | 1.5 |
| Dextrin fatty acid coated black iron oxide | 0.5 |
| Purified water | Balance |
| Total | 100.00 |

## Claims

1. A water-in-oil emulsified cosmetic comprising the following components (a) to (d):
(a) one or more polymers selected from the group consisting of polyacrylic acid, a salt of polyacrylic acid, poly(2-acrylamido-2-methylpropanesulfonic acid), and a salt of poly(2-acrylamido-2-methylpropanesulfonic acid) and
having a weight average molecular weight of 1 million to 4 million, and
a viscosity at 25°C when made into a 1% by mass aqueous solution of 1 Pa·s or less,
(b) an aqueous phase component other than the component (a)
(c) 60% by mass or less of an oily component, and
(d) an emulsifier.

2. The water-in-oil emulsified cosmetic according to claim 1, wherein a content of the component (a) is 0.005% to 2%.

3. The water-in-oil emulsified cosmetic according to claim 1 or 2, wherein a weight average molecular weight of the component (a) is 1.5 million to 4 million.

4. The water-in-oil emulsified cosmetic according to any one of claims 1 to 3, further comprising a hydrophobized powder as a component (e).
